(19) Europäisches Patentamt
European Patent Office
Office européen des brevets

(11) Veröffentlichungsnummer: **0 226 724**
**B1**

(12) **EUROPÄISCHE PATENTSCHRIFT**

(45) Veröffentlichungstag der Patentschrift: **25.01.89**

(51) Int. Cl.⁴: **C07C 102/08**, C07C 103/133

(21) Anmeldenummer: **86113118.3**

(22) Anmeldetag: **24.09.86**

(54) **Verfahren zur Herstellung von Methacrylamid.**

(30) Priorität: **22.11.85 DE 3541253**

(43) Veröffentlichungstag der Anmeldung:
**01.07.87 Patentblatt 87/27**

(45) Bekanntmachung des Hinweises auf die Patenterteilung:
**25.01.89 Patentblatt 89/4**

(84) Benannte Vertragsstaaten:
**DE FR GB IT**

(56) Entgegenhaltungen:
**DE-B- 1 283 220**
**DE-B- 1 618 721**
**GB-A- 1 186 876**
**US-A- 3 301 900**
**US-A- 4 521 620**

**CHEMICAL ABSTRACTS, Band 82, Nr. 20, 19. Mai 1975, Seite 14, Abstract Nr. 125795n, Columbus, Ohio, US; K. TAGAKI et al.: "Methacrylamide sulfate"; & JP - A - 49 109 314 (SUMITOMO CHEMICAL CO.) 17.10.1974**

(73) Patentinhaber: **Degussa Aktiengesellschaft, Weissfrauenstrasse 9, D-6000 Frankfurt am Main 1(DE)**

(72) Erfinder: **Rudolph, Udo, Ing., Grünaustrasse 11, D-6450 Hanau 9(DE)**
Erfinder: **Möller, Lothar, Dr. Dipl.-Ing., Steinheimer Strasse 85, D-6453 Seligenstadt(DE)**
Erfinder: **Meiers, Horst, Chem.-Ing., Fritz-Schubert-Ring 46, D-6454 Bruchköbel(DE)**

**EP 0 226 724 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Herstellung von Methacrylamid aus Acetoncyanhydrin (ACH) und konzentrierter Schwefelsäure.

Methacrylamid ist ein wichtiges Zwischenprodukt bei der Herstellung von Methacrylsäureestern für Kunststoffe und Kunststoffadditive. Man verwendet heute allgemein die von Crawford entwickelte Methode (US-PS 2 042 458).

Die Synthese von Methacrylamid aus Acetoncyanhydrin in Gegenwart von konzentrierter Schwefelsäure bzw. Oleum verläuft nach allgemeiner Ansicht in zwei Schritten.

In dem ersten Schritt entstehen aus dem Acetoncyanhydrin und der Schwefelsäure bei 60 bis 100°C gleichzeitig α-Hydroxyisobutyramid und dessen saures Sulfat:

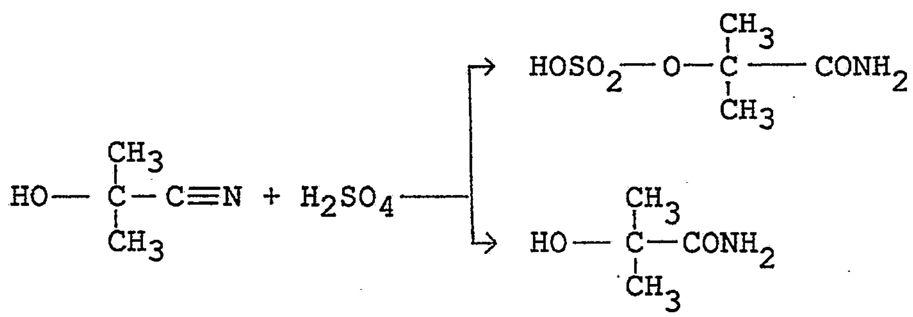

In dem zweiten Schritt wird das saure Sulfat bei Erwärmen des Gemisches auf 130 bis 160°C quantitativ zu Methacrylamid konvertiert :

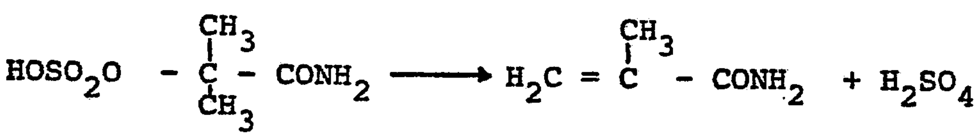

Das neben seinem sauren Sulfat entstandene freie α-Hydroxyisobutyramid wird hingegen nur teilweise in Methacrylamid umgewandelt. Der Umsetzungsgrad zu α-Hydroxyisobutyramid sowie seine Konvertierung zu Methacrylamid hängen stark vom Molverhältnis Schwefelsäure zu Acetoncyanhydrin ab.

In den Angaben über die technischen Bedingungen bei der Herstellung von Methacrylamid findet man für die Amidierungsreaktion häufig unterschiedliche Daten. Als optimal für die Amidierung werden aber die nachstehenden Bedingungen angesehen:

Molverhältnis Schwefelsäure (bzw. Oleum) zu Acetoncyanhydrin = 1:1 bis 2:1, unter der Voraussetzung, daß die Schwefelsäurekonzentration nicht unter 98% liegen darf, und die Temperatur zwischen 100 und 150°C liegt.

Nach Polievka, M; Uhlar, L. (Petrochemisches Forschungsinstitut Novaky) Letz, S. (PCHZ, Zilina); Petrochémia 22 (1982) 2; 33–38) die in einem Übersichtsartikel über den Stand der Technik berichten, liegt für einen Batchprozeß der optimale Wert für den Schwefelsäureüberschuß bei 1,4 bis 1,6.

Die Schwierigkeiten bei der Durchführung dieser Reaktion rühren vor allem aus der hohen Reaktionsenthalpie der Umsetzung von ACH und Schwefelsäure und aus der hohen Viskosität der Reaktionsmischung bei abnehmendem Schwefelsäureüberschuß.

Man kann theoretisch zwar mit geringen Überschüssen an Schwefelsäure arbeiten, muß aber in diesem Fall für die Umwälzung des Reaktionsgemisches zur Abfuhr der Reaktionsenthalpie und zum Durchmischen der Reaktanden große Mengen an Energie aufbringen, da mit sinkendem Schwefelsäureüberschuß die Viskosität des Gemisches erheblich ansteigt.

Ein Ausweg aus dieser Situation wird in der DE-PS 1 618 721 vorgeschlagen, ohne aber auf die erforderliche Mischgüte beim Zusammenführen der Reaktanden einzugehen.

Gemäß dieser Patentschrift wiederholt man Umsetzung und Konvertierung in dieser Reihenfolge mindestens einmal, setzt dabei in der ersten Stufe einen hohen Überschuß an Schwefelsäure ein, und fügt in jeder folgenden ACH zu, um das Molverhältnis von eingesetzter Schwefelsäure zu ACH dem Wert 1:1 anzunähern. Eine Stufe umfaßt jeweils Umsetzung und Konvertierung.

Auf diesem Wege erreicht man zwar eine Verringerung des Schwefelsäureverbrauchs, muß aber immer noch hohe Umwälzleistungen aufbringen und sich mit einer Ausbeute von max. 93% (einschließlich der gebildeten Methacrylsäure) zufriedengeben.

Aufgabe der Erfindung ist ein Verfahren zur Herstellung von Methacrylamid (MAA), bei dem die aufzuwendende Umwälzleistung erheblich verkleinert, und das sowohl kontinuierlich als auch diskontinuierlich mit guten Ausbeuten betrieben werden kann.

Gegenstand der Erfindung ist ein Verfahren zur Herstellung von Methacrylamid durch Umsetzung von Acetoncyanhydrin (ACH) mit konzentrierter Schwefelsäure und Konvertierung der dabei entstehenden Zwischenprodukte bei erhöhter Temperatur, dadurch gekennzeichnet, daß man ein zweiphasiges Gemisch (Emulsion), bestehend aus einem inerten geradkettigen gesättigten Kohlenwasserstoff mit 5 bis 7 Kohlenstoffatomen und ACH, in die Schwefelsäure einleitet.

Die Konzentration der eingesetzten Schwefelsäure beläuft sich auf mindestens 97,5 Gew.-%, vorzugsweise 100 Gew.-%. Als Kohlenwasserstoff setzt man bevorzugt n-Hexan in einem molaren Verhältnis von ca. 3:1, bezogen auf ACH ein. Das bedeutet aber nicht, daß das Gemisch Hexan/ACH bei der Einspeisung immer diese Zusammensetzung hat. Dieser Wert gilt für die Gesamtbilanz, während man über das aktuelle Einspeiseverhältnis die Reaktionstemperatur steuert.

In Abhängigkeit davon setzt man in einer anderen Ausführungsform bis zu 20% Hexan mehr bzw. bis zu 50% weniger Hexan als durch das Molverhältnis von 3:1 vorgegeben, zu.

Die Umsetzungstemperatur läßt sich über die Zugabe des Kohlenwasserstoffs genau steuern, dessen Dampf kondensiert und anschließend mit weiterem ACH vermischt wieder der Reaktion zugeführt wird.

Kohlenwasserstoff und ACH werden vor der Eindosierung intensiv z.B. in einem Statikmischer durchmischt, ohne daß dabei eine bestimmte Temperatur vorgeschrieben wäre.

In der Praxis stammt der Kohlenwasserstoff aus der Destillatrückgewinnung des Reaktionskreislaufes, so daß seine Temperatur im allgemeinen ca. 20 bis 30°C unterhalb seines Siedepunktes liegt.

Dagegen wird das ACH, das bei 0 bis 20°C gelagert wird, bei diesen Temperaturen mit dem Kohlenwasserstoff vermischt.

Die Umsetzung von ACH und Schwefelsäure führt man so, daß am Schluß der Reaktion das molare Verhältnis von eingesetzter Schwefelsäure zu insgesamt eingesetztem ACH > 1,1 bis ≤ 1,4:1 beträgt. Bevorzugt wird ein Verhältnis von eingesetzter Schwefelsäure zu eingesetztem ACH von 1,4 angestrebt.

Das erfindungsgemäße Verfahren kann sowohl diskontinuierlich als auch mehrstufig kontinuierlich durchgeführt werden, wobei auch Mischformen möglich sind. Bei diskontinuierlicher Betriebsweise setzt man das ACH/Hexan-Gemisch z.B. über eine Ringbrause innerhalb von 3 bis 120 min., bevorzugt 30 bis 60 min, der auf die Reaktionstemperatur aufgeheizten Schwefelsäure bzw. dem Reaktionsgemisch zu. Dabei ist, je nach Batchzeit oder mittlerer Verweilzeit in den einzelnen Reaktoren bei mehrstufig kontinuierlicher Betriebsweise, die Reaktionstemperatur in Abhängigkeit vom eingesetzten Schwefelsäure/ACH-Verhältnis so zu steuern, daß sie mit abnehmendem Schwefelsäure/ACH-Verhältnis ansteigt.

Für die Einmischung des ACH/Hexan-Gemisches in die Schwefelsäure bzw. das Reaktionsgemisch sind besonders dynamische Mischer z.B. nach dem Rotor-Stator-Prinzip geeignet.

Mit dem Anspringen der Reaktion setzt aufgrund der freiwerdenden Reaktionsenthalpie die Verdampfung des Kohlenwasserstoffs ein. Die damit verbundene Blasenbildung führt zu einer starken Durchmischung der Reaktanden.

Die so nutzbare Verdrängungsarbeit der Blasen liegt mindestens in der gleichen Größenordnung wie die Mischleistung eines Rührers oder Statikmischers und ermöglicht erstmals auch im technischen Maßstab eine ökonomisch sinnvolle Absenkung des molaren Schwefelsäureüberschusses auf Werte von > 1,1 bis ≤ 1,4:1, bevorzugt von 1,15:1, bezogen auf ACH.

Die Zugabe des Kohlenwasserstoffes wird so gesteuert, daß nach Beendigung der Umsetzung das Reaktionsgemisch diese Verbindung nicht mehr enthält.

Da bei den geringen Schwefelsäureüberschüssen die Viskosität der Reaktionsmischung mit zunehmender ACH-Zugabe ansteigt, ist es vorteilhaft, die Temperatur während der Umsetzung in Abhängigkeit von der Viskositätszunahme von den an sich bekannten 60°C bis 90° auf 100° bis 130°C, anzuheben. Dabei ist die Reaktionszeit so zu wählen, daß keine vollständige Konvertierung eintritt.

Beim Batchbetrieb kühlt man nach Abschluß der Umsetzung schnell, bevorzugt innerhalb von 3 bis 15 min auf 90° bis 100°C ab und führt anschließend bei den an sich bekannten Temperaturen von 130 bis 160°C die Restkonvertierung zum Methacrylamid kontinuierlich durch.

Man erhält auf diesem Wege z.B. Ausbeuten von 94,0% mit einem Molverhältnis Schwefelsäure/ACH von 1,15:1. Dies überrascht, da nach dem Stand der Technik angenommen werden mußte, daß oberhalb von 100°C die Zersetzung von ACH zu Ausbeuteverlusten führt.

Das beschriebene Verfahren kann aber auch kontinuierlich durchgeführt werden. Hierbei ist z.B.denken, daß die Ausbeuten eines Batchprozesses nur durch unendlich viel hintereinandergeschaltete Stufen eines kontinuierlichen Prozesses mit angepaßter ACH-Einspeisung in jeder Stufe realisiert werden können. Nur so werden die für eine optimale Ausbeute notwendigen Molverhältnisse von Schwefelsäure zu ACH von ∞ bis zum angestrebten Endwert durchlaufen. Aus diesem Grund sollte bei einem kontinuierlichen Prozeß grundsätzlich mehrstufig gearbeitet werden. Bei einer beispielsweise zweistufigen, kontinuierlichen Betriebsweise wird wie folgt gearbeitet:

In der ersten Stufe setzt man ACH/Hexan-Gemisch mit Schwefelsäure in einem Molverhältnis von mindestens 1,5:1 (Schwefelsäure/ACH), bevorzugt 1,5:1 bis 2,0:1, um und konvertiert anschließend teilweise zum Methacrylamid.

Dieses Reaktionsgemisch versetzt man nach dem Abkühlen auf 90 bis 100°C erneut mit einem solchen Anteil des ACH/Hexan-Gemisches, daß sich das Molverhältnis der insgesamt eingesetzten Reaktanden Schwefelsäure und ACH auf > 1,1 bis ≤ 1,4, bevorzugt 1,4 absenkt.

Bei Vergleichsversuchen im kontinuierlichen Betrieb (zweistufige Fahrweise) ergab sich, daß man bei einem molaren Schwefelsäure/ACH-Verhältnis von 1,4:1 in der zweiten Stufe ohne Hexan-Zusatz eine Antriebsleistung von 300 kW für die Umwälzpumpen benötigt, während nach dem erfindungsgemäßen Verfahren unter Hexanzusatz eine Leistung von nur 50 kW ausreicht.

Obwohl in beiden Fällen die Umsetzungstemperaturen (100°C) und die Konvertierungstemperaturen (140°C) gleich waren, ergab sich erfindungsgemäß zusätzlich noch eine Ausbeutesteigerung um 1% von 92,5% auf 93,5% im Vergleich mit dem bekannten Verfahren.

In einer vorteilhaften Ausführungsform des erfindungsgemäßen Verfahrens kombiniert man die ansatzweise durchgeführte Umsetzung mit der kontinuierlich ablaufenden Konvertierung und gelangt so zu Ausbeuten von fast 95% bei einem Molverhältnis von Schwefelsäure zu ACH von 1,4:1, sowie zu 94% Ausbeute bei einem Molverhältnis von 1,15:1.

Für die sich anschließende kontinuierliche Konvertierung sind die angewandten Temperaturen und Verweilzeiten der Menge der überschüssigen Schwefelsäure anzupassen. Als Konvertierungszeit sind 20 bis 700 sec. anzusetzen. Dabei entsprechen den kürzeren Zeiten die höheren Temperaturen und die größeren Schwefelsäureüberschüsse.

Bei Vergleichsversuchen mit gleicher Apparatur und Mischsystem, aber ohne Verdampfungskühlung (ohne Hexaneinsatz), sondern indirekter Kühlung, ergaben sich bei gleichen Molverhältnissen Schwefelsäure zu ACH wesentlich geringere Ausbeuten und zwar von 93% bei einem Verhältnis von 1,4:1 und 91% bei einem Verhältnis von 1,15:1.

<u>Versuchsdurchführung für kontinuierliche Betriebsweise</u>

Die Anlage, Bild 1, wird wie folgt betrieben:

2/3 des insgesamt – bezogen auf den angestrebten $H_2SO_4$-Überschuß – zu dosierenden ACH werden zusammen mit der erforderlichen Menge n-Hexan als Kühlmedium über eine Ringbrause (Kranz feiner Austrittsöffnungen nach unten gerichtet) mit Dosierpumpen in den 1. Reaktor gefördert. In die Mitte der Ringbrause des 1. Reaktors wurde die gesamte Oleummenge dosiert. In einem Abstand von 0,5 mm dreht sich unter der Ringbrause ein Flügelrührer mit ca. 2500 U/min, der die beiden Reaktanden spontan in das Reaktionsgemisch (Reaktionsvolumen ca. 300 ml) einarbeitet. Die Reaktionsenthalpie wird durch Verdampfen des n-Hexan abgeführt, wobei die gewünschte Reaktionstemperatur durch die Dosiermenge an n-Hexan genau eingestellt werden kann. Das n-Hexan wird im Kreis gefahren, d. h. kondensiert und in den Reaktor zurückgeführt. Das Volumen des Reaktionsgemisches ist durch einen Siphon bestimmt. Es läuft auf einer seitlich am Boden des Reaktors befindlichen Öffnung durch ein entsprechend hochgeführtes Überlaufrohr zur Konvertierung zunächst als Rieselfilm von oben in einen Fallfilmverdampfer, der eine schnelle Aufheizung auf die Konvertierungstemperatur bewirkte. Von dort wird das Reaktionsgemisch durch eine Konvertierungsstrecke – in Form eines beheizten Rohres – geführt.

Das MAA-$H_2SO_4$-Gemisch läuft zur schnellen Abkühlung in einen Pufferbehälter mit siedendem n-Hexan ein und wird als untere Phase über den Bodenablauf des Behälters mit einer Kolbenpumpe in die Mitte der Ringbrause des zweiten Reaktors gefördert. Das restliche Drittel der ACH-Menge wird zusammen mit n-Hexan über die Ringbrause eingebracht und wieder durch einen Flügelrührer spontan eingearbeitet. Die anschließende Konvertierung erfolgt wie in der ersten Stufe. Beide Reaktoren sind baugleich. Sämtliche produktführenden Rohrleitungen sind begleitbeheizt.

Die Anlage kann auch einstufig betrieben werden:

dann erfolgt die Dosierung der Gesamtmenge ACH bereits im ersten Reaktor oder zweistufig wie beschrieben. Im beschriebenen Fall sollte die Konvertierungszeit der ersten Stufe, bezogen auf die vorgegebene Konvertierungstemperatur, so gewählt werden, daß sie gerade etwa 20% der optimalen, für das Erreichen des Ausbeutemaximums erforderlichen, entspricht. Die optimale Konvertierungszeit ermittelt man durch Vorversuche.

<u>Beispiele zu kontinuierlicher Fahrweise</u>

Beispiel 1. einstufig
Molverhältnis $H_2SO_4$:ACH = 2,0
Reaktionstemperatu r= 90°C
Konvertierungstemperatur = 140°C
Konvertierungszeit = 1,8 min
Amidausbeute = 95,1%

Beispiel 2. einstufig
Molverhältnis $H_2SO_4$:ACH = 1,4
Reaktionstemperatur = 100°C

Konvertierungstemperatur = 150°C
Konvertierungszeit = 3,8 min
Amidausbeute = 93,1%

Beispiel 3. einstufig
Molverhältnis $H_2SO_4$:ACH = 1,15
Reaktionstemperatur = 110°C
Konvertierungstemperatur = 160°C
Konvertierungszeit = 3,8 min
Amidausbeute = 89,8%

Beispiel 4. zweistufig

1. Stufe

Molverhältnis $H_2SO_4$:ACH = 2,0
Reaktionstemperatur = 90°C
Konvertierungstemperatur = 140°C
Konvertierungszeit = 0,4 min

2. Stufe

Molverhältnis $H_2SO_4$:ACH = 1,4
Reaktionstemperatur = 100°C
Konvertierungstemperatur = 150°C
Konvertierungszeit = 3,8 min
Amidausbeute = 93,8%

Beispiel 5. zweistufig

1. Stufe

Molverhältnis $H_2SO_4$:ACH = 1,73
Reaktionstemperatur = 100°C
Konvertierungstemperatur = 140°C
Konvertierungszeit = 0,7 min

2. Stufe

Molverhältnis $H_2SO_4$:ACH = 1,15
Reaktionstemperatur = 110°C
Konvertierungstemperatur = 160°C
Konvertierungszeit = 3,8 min
Amidausbeute = 90,7%

<u>Versuchsdurchführung für Batchfahrweise</u>

Die Anlage, Bild 2, wird wie folgt betrieben:

In einem gegebenenfalls über den Mantel kühl- oder beheizbaren Glasreaktor wird 100%ige $H_2SO_4$ vorgelegt. ACH im Gemisch mit n-Hexan wird über eine Ringbrause, die in die Schwefelsäure eintaucht, dosiert. Unter der Ringbrause dreht ein Flügelrührer mit max. 2500 $min^{-1}$. Das verdampfende Hexan wird kondensiert und mit neuem ACH wieder in die Reaktion zurückgeführt. Nach Beendigung der ACH-Dosierung wird die Reaktionsmischung zur Konvertierung durch eine beheizte Rohrleitung geführt.

Die Reaktionstemperatur ist entsprechend der Dosiergeschwindigkeit des ACH, d.h. entsprechend der Batchzeit sowie dem abnehmenden $H_2SO_4$/ACH-Verhältnis zu steigern (bei unseren Versuchen linear von der Zeit) und zwar durch Regelung der Hexanzufuhr.

Beispiele zur Batchfahrweise

Beispiel 1. mit Verdampfungskühlung

vorgelegte Oleummenge = 1200 g
dosierte ACH-Menge 98%ig = 925 g
Molverhältnis $H_2SO_4$/ACH = 1,15
Dosierzeit ACH = 7 min
Reaktionstemperatur
Anfang = 70°C
Ende = 130°C
Konvertierungstemperatur = 150°C
Konvertierungszeit = 6,8 min
Amidausbeute = 93,9%

Bemerkung:

Vor der Konvertierung wurde das Reaktionsgemisch durch über die ACH-Dosierzeit hinaus verlängerte Hexanzugabe auf 95°C abgekühlt.

Beispiel 2. mit Verdampfungskühlung

vorgelegte Oleummenge = 1200 g
dosierte ACH-Menge 98%ig = 925 g
Molverhältnis $H_2SO_4$/ACH = 1,15
Dosierzeit ACH = 60 min
Reaktionstemperatur
Anfang = 70°C
Ende = 110°C
Konvertierungstemperatur = 160°C
Konvertierungszeit = 3,7 min
Amidausbeute = 94,0%

Beispiel 3. ohne Verdampfungskühlung (Mantelkühlung)

vorgelegte Oleummenge = 1200 g
dosierte ACH-Menge 98%ig = 925 g
Molverhältnis $H_2SO_4$/ACH = 1,15
Dosierzeit ACH = 60 min
Reaktionstemperatur
Anfang = 70°C
Ende = 110°C
Konvertierungstemperatur = 160°C
Konvertierungszeit = 3,7 min
Amidausbeute = 91,1%

Beispiel 4. mit Verdampfungskühlung

vorgelegte Oleummenge = 1200 g
dosierte ACH-Menge 98%ig = 760 g
Molverhältnis $H_2SO_4$/ACH = 1,4
Dosierzeit ACH = 8 min
Reaktionstemperatur
Anfang = 70°C
Ende = 90°C
Konvertierungstemperatur = 150°C
Konvertierungszeit = 3,7 min
Amidausbeute = 95,1%

## Patentansprüche

1. Verfahren zur Herstellung von Methacrylamid durch Umsetzung von Acetoncyanhydrin (ACH) mit konzentrierter Schwefelsäure und Konvertierung des dabei entstehenden Zwischenprodukts bei erhöhter Temperatur, dadurch gekennzeichnet, daß man ein zweiphasiges flüssiges Gemisch, bestehend aus einem inerten, geradkettigen gesättigten Kohlenwasserstoff mit 5 bis 7 Kohlenstoffatomen und ACH, in die Schwefelsäure einleitet.

2. Verfahren nach Anspruch 1, dadurch gekennzeichnet, daß man n-Hexan in einem molaren Verhältnis von 3 (bis +20% und bis –50%):1 bezogen auf ACH, entsprechend der abzuführenden Reaktionsenthalpie und der gewünschten Reaktionstemperatur, einsetzt.

3. Verfahren nach den Ansprüchen 1 und 2, dadurch gekennzeichnet, daß man der Schwefelsäure das ACH/Hexan-Gemisch zusetzt, bis ein Molverhältnis Schwefelsäure/ACH von > 1,1:1 bis ≤ 1,4:1 erreicht ist.

4. Verfahren nach den Ansprüchen 1 bis 3, dadurch gekennzeichnet, daß man bei diskontinuierlicher Betriebsweise die Umsetzungstemperatur in Abhängigkeit vom Anstieg der Viskosität und Abhängigkeit von der Art des verwendeten Kohlenwasserstoffs von 60°C auf 130°C anhebt, und nach Abschluß der Umsetzung schnell auf 90°C bis 100°C abkühlt.

5. Verfahren nach den Ansprüchen 1 und 2, dadurch gekennzeichnet, daß man bei kontinuierlicher Betriebsweise die Umsetzung und Konvertierung mindestens einmal wiederholt und bei jeder folgenden Umsetzung ein ACH/Hexan-Gemisch zusetzt, und das Molverhältnis Schwefelsäure/ACH auf den Wert von > 1,1 bis ≤ 1,4:1 einstellt, wobei die Zwischenkonvertierung nicht vollständig durchgeführt wird.

## Claims

1. Process for the preparation of methacrylamide by reacting acetone cyanohydrin (ACH) with concentrated sulphuric acid, and converting the intermediate produced in this reaction at elevated temperature, characterized in that a two-phase liquid mixture comprising an inert, straight-chain saturated hydrocarbon having 5 to 7 carbon atoms and ACH, is introduced into the sulphuric acid.

2. Process according to claim 1, characterized in that n-hexane is employed in a molar ratio of 3 (up to +20% and up to –50%):1, relative to ACH, corresponding to the reaction enthalpy to be dissipated and the reaction temperature desired.

3. Process according to claims 1 and 2, characterized in that the ACH/hexane mixture is added to the sulphuric acid until a sulphuric acid/ACH molar ratio of > 1.1:1 to ≤ 1.4:1 is reached.

4. Process according to claims 1 to 3, characterized in that, in a batchwise procedure, the reaction temperature is increased from 60°C to 130°C as a function of the increase in viscosity and as a function of the type of hydrocarbon used, and the reaction mixture is cooled rapidly to between 90°C and 100°C when the reaction is complete.

5. Process according to claims 1 and 2, characterized in that, in a continuous procedure, the reaction and conversion are repeated at least once, and an ACH/hexane mixture is added in each subsequent reaction, and the sulphuric acid/ACH molar ratio is set to the value > 1.1 to ≤ 1.4:1 , the intermediate conversion not being carried out to completion.

## Revendications

1. Procédé de préparation de méthacrylamide par réaction d'acétone, cyanhydrine (ACH) avec de l'acide sulfurique concentré et conversion du produit intermédiaire ainsi formé à haute température, caractérisé en ce qu'on introduit dans l'acide sulfurique un mélange liquide biphasé constitué d'un hydrocarbure saturé linéaire avec 5 à 7 atomes de carbone et d'ACH.

2. Procédé selon la revendication 1, caractérisé en ce qu'on utilise le n-hexane en rapport molaire de 3 (à +20% et jusqu'à –50%):1 rapporté à l'ACH, suivant l'enthalpie de réaction à évacuer et la température de réaction souhaitée.

3. Procédé selon les revendications 1 et 2, caractérisé en ce qu'on ajoute à l'acide sulfurique le mélange ACH/hexane jusqu'à ce qu'on obtienne un rapport molaire acide sulfurique/ACH > 1,1:1 jusqu'à ≤ 1,4:1.

4. Procédé selon l'une des revendications 1 à 3, caractérisé en ce qu'en fonctionnement en discontinu, on élève la température de réaction de 60°C à 130°C en fonction de l'augmentation de la viscosité et en fonction du type d'hydrocarbure utilisé, et à la fin de la réaction, on refroidit rapidement à 90°C–100°C.

5. Procédé selon les revendications 1 et 2, caractérisé en ce qu'en fonctionnement en continu, la réaction et la conversion sont répétées au moins une fois, et que pour toute réaction suivante, on ajoute un mélange ACH/hexane, et on règle le rapport molaire acide sulfurique/ACH de la valeur de > 1,1 à ≤ 1,4:1, la conversion intermédiaire n'étant pas réalisée complètement.

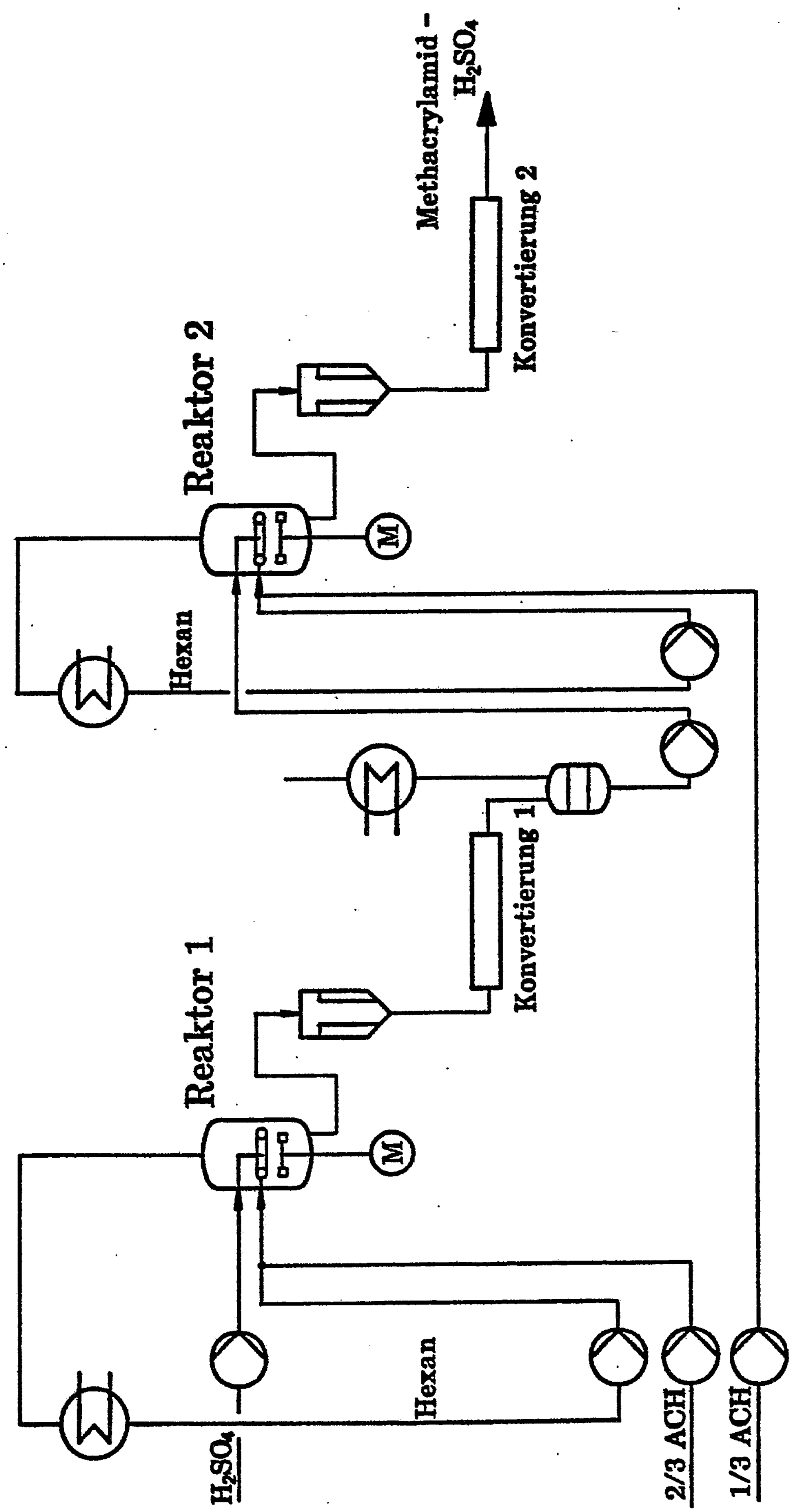
Methacrylamid -
$H_2SO_4$
Konvertierung 2
Reaktor 2
M
Hexan
Konvertierung 1
Reaktor 1
M
Hexan
$H_2SO_4$
2/3 ACH
1/3 ACH

Bild 1

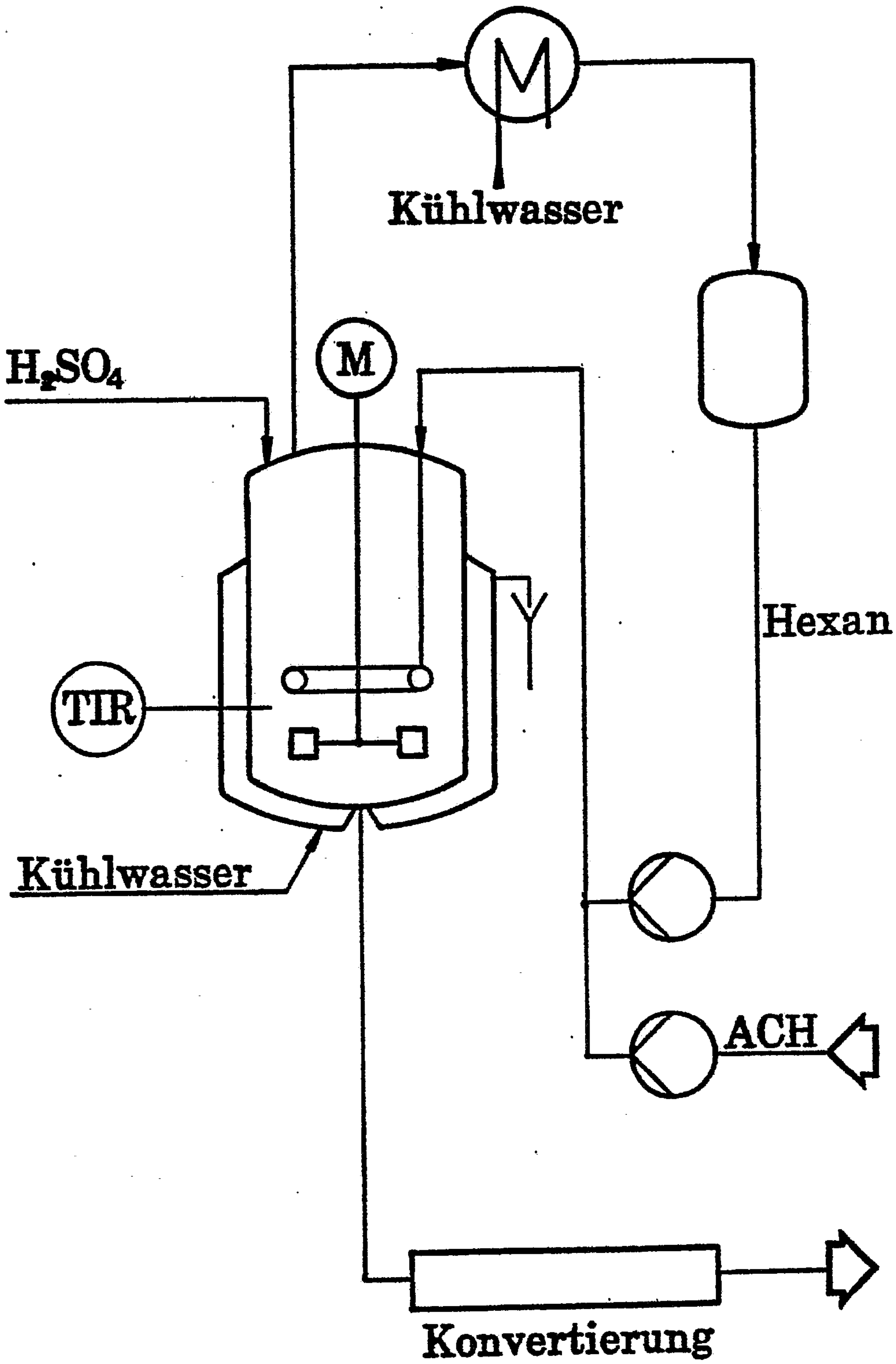
Kühlwasser
$H_2SO_4$
M
Hexan
TIR
Kühlwasser
ACH
Konvertierung

Bild 2